(19) Europäisches Patentamt
European Patent Office
Office européen des brevets

(11) Veröffentlichungsnummer: **0 365 833 B1**

(12) **EUROPÄISCHE PATENTSCHRIFT**

(45) Veröffentlichungstag der Patentschrift: **06.07.94**

(21) Anmeldenummer: **89117556.4**

(22) Anmeldetag: **22.09.89**

Die Akte enthält technische Angaben, die nach dem Eingang der Anmeldung eingereicht wurden und die nicht in dieser Patentschrift enthalten sind.

(51) Int. Cl.5: **C07D 261/04**, A01N 43/74, C07D 413/04, C07D 333/22, C07D 409/04, C07D 417/04, C07D 307/52, C07D 409/14, C07D 413/14, C07F 9/653, C07F 7/18

# (54) Cyclohexen-Derivate.

(30) Priorität: **30.09.88 DE 3833264**

(43) Veröffentlichungstag der Anmeldung:
**02.05.90 Patentblatt 90/18**

(45) Bekanntmachung des Hinweises auf die Patenterteilung:
**06.07.94 Patentblatt 94/27**

(84) Benannte Vertragsstaaten:
**BE CH DE ES FR GB IT LI NL**

(56) Entgegenhaltungen:
**EP-A- 0 103 143**
**EP-A- 0 125 094**
**EP-A- 0 136 702**
**EP-A- 0 162 224**
**EP-A- 0 177 913**
**EP-A- 0 232 724**
**EP-A- 0 238 021**

(73) Patentinhaber: **BASF Aktiengesellschaft**
**Carl-Bosch-Strasse 38**
**D-67063 Ludwigshafen(DE)**

(72) Erfinder: **Kolassa, Dieter, Dr.**
**Moltkestrasse 8**
**D-6700 Ludwigshafen(DE)**
Erfinder: **Kast, Juergen, Dr.**
**Kastanienstrasse 24**
**D-6737 Boehl-Iggelheim(DE)**
Erfinder: **Kuekenhoehner, Thomas, Dr.**
**Seidelstrasse 2**
**D-6710 Frankenthal(DE)**
Erfinder: **Meyer, Norbert, Dr.**
**Dossenheimer Weg 22**
**D-6802 Ladenburg(DE)**
Erfinder: **Westphalen, Karl-Otto, Dr.**
**Mausbergweg 58**
**D-6720 Speyer(DE)**
Erfinder: **Wuerzer, Bruno, Dr.**
**Ruedigerstrasse 13**
**D-6701 Otterstadt(DE)**

**EP 0 365 833 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Cyclohexenonderivate der allgemeinen Formel I

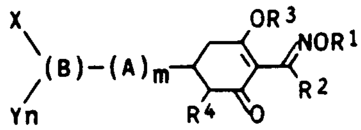

I

in der die Substituenten folgende Bedeutung haben:

- $R^1$ eine $C_1$-$C_6$-Alkylgruppe, eine $C_3$-$C_6$-Alkenylgruppe oder eine $C_3$-$C_6$-Alkinylgruppe, wobei diese Gruppen ein bis drei der folgenden Reste tragen können: Halogen, $C_1$-$C_4$-Alkoxy, $C_1$-$C_4$-Alkylthio und/oder Phenyl, wobei die Phenylgruppe ihrerseits bis zu dreifach durch Halogen, $C_1$-$C_4$-Alkyl, $C_1$-$C_4$-Alkoxy, $C_1$-$C_4$-Alkylthio, $C_1$-$C_4$-Halogenalkyl und/oder Cyano substituiert sein kann;
  eine Phenylgruppe, eine Benzylgruppe oder eine Thenylgruppe, wobei die aromatischen Kerne ein bis drei der folgenden Reste tragen können: Halogen, $C_1$-$C_4$-Alkyl, $C_1$-$C_4$-Alkoxy, $C_1$-$C_4$-Alkylthio, $C_1$-$C_4$-Halogenalkyl, Nitro und/oder Cyano;
- $R^2$ eine $C_1$-$C_6$-Alkylgruppe, eine $C_3$-$C_6$-Alkenylgruppe, eine $C_3$-$C_6$-Alkinylgruppe oder eine Phenylgruppe, wobei diese Gruppen ein bis drei der folgenden Reste tragen können: Halogen, $C_1$-$C_4$-Alkoxy, $C_1$-$C_4$-Alkylthio und/oder Cyano;
- $R^3$ Wasserstoff, eine $C_1$-$C_6$-Alkylgruppe, eine $C_1$-$C_{10}$-Alkylcarbonylgruppe, eine $C_1$-$C_6$-Alkylsulfonylgruppe, eine $C_1$-$C_4$-Trialkylsilylgruppe, eine $C_1$-$C_4$-Dialkylphosphonylgruppe, eine $C_1$-$C_4$-Dialkylthiophosphonylgruppe, eine $C_3$-$C_6$-Alkenylgruppe, eine $C_3$-$C_6$-Alkenylcarbonylgruppe, eine $C_3$-$C_6$-Alkinylgruppe oder
  eine Benzoyl- oder Phenylsulfonylgruppe, die ein- bis dreifach durch Halogen, Nitro, Cyano, $C_1$-$C_4$-Alkyl, $C_1$-$C_4$-Halogenalkyl, $C_1$-$C_4$-Alkoxy und/oder $C_1$-$C_4$-Alkylthio substituiert sein können;
- $R^4$ Wasserstoff, eine Cyanogruppe, ein Halogenatom, eine $C_1$-$C_6$-Alkylgruppe oder eine $C_1$-$C_6$-Alkoxycarbonylgruppe;
- A eine $C_1$-$C_4$-Alkylenkette, in der eine Methylengruppe durch ein Sauerstoff- oder Schwefelatom ersetzt sein kann und die ein bis zwei der folgenden Substituenten tragen kann: $C_1$-$C_4$- Alkyl, $C_1$-$C_4$-Alkoxy, $C_1$-$C_4$-Alkylthio und/oder Halogen;
- m 0 oder 1
- B ein 5-gliedriger einfach oder doppelt ungesättigter Heterocyclus mit bis zu 2 Stickstoffatomen und/oder bis zu einem Sauerstoff- oder Schwefelatom;
- X ein 5 - 6-gliedriger Heteroaromat, wobei diese Ringe ein bis drei der folgenden Gruppen tragen können: $C_1$-$C_4$-Alkyl, $C_1$-$C_4$-Alkoxy, $C_1$-$C_4$-Alkylthio, $C_1$-$C_4$-Halogenalkyl, Halogen, Cyano und/oder Nitro;
- Y Wasserstoff, ein Halogenatom, eine Cyanogruppe, eine Nitrogruppe;
  eine $C_1$-$C_6$-Alkylgruppe, eine $C_2$-$C_6$-Alkenylgruppe oder eine $C_2$-$C_6$-Alkinylgruppe, wobei diese Gruppen ein bis drei der folgenden Reste tragen können: Phenyl, Halogen, $C_1$-$C_4$-Alkoxy und/oder $C_1$-$C_4$-Alkylthio,
  eine $C_1$-$C_4$-Alkoxygruppe, eine $C_1$-$C_4$-Alkylthiogruppe, eine $C_3$-$C_6$-Alkenyloxygruppe oder eine $C_3$-$C_6$-Alkinyloxygruppe;
- n 1, 2 oder 3,

sowie deren umweltverträglichen Salze.

Außerdem betrifft die Erfindung die Herstellung der Verbindungen I, deren Verwendung als Herbizide sowie Mischungen zur Bekämpfung unerwünschten Pflanzenwuchses, welche die Verbindungen der Formel I enthalten.

Die Verbindungen der Formel I können in mehreren tautomeren Formen vorliegen, die alle vom Patentanspruch umfaßt werden und sich im Fall von $R^3$ = H wie folgt darstellen lassen:

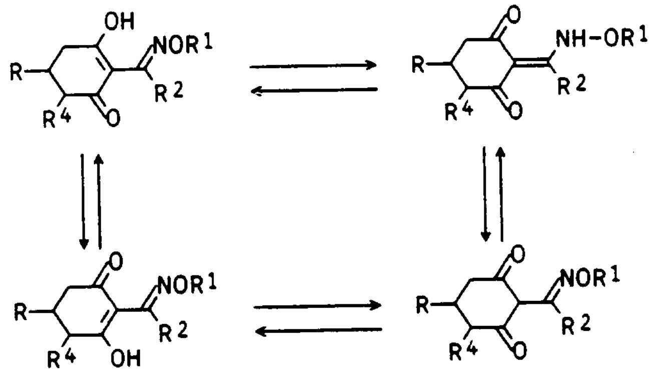

In diesem Schema wie in allen folgenden Reaktionsgleichungen ist der Rest

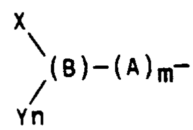

zur besseren übersichtlichkeit mit R bezeichnet.

Die herbizide Wirkung von 3-Hydroxy-2-cyclohexen-1-on-oximetherderivaten,die in 5-Position einen 5-gliedrigen heterocyclischen Rest tragen, ist aus der EP-A 125 094, der EP-A 162 224 und der EP-A 238 021 bekannt.

Es werden jedoch Verbindungen gesucht, die bei geringeren Aufwandmengen eine bessere Verträglichkeit (Selektivität) mit Kulturpflanzen bei guter Wirkung gegen Schadpflanzen aufweisen.

Entsprechend dieser Aufgabe wurden die eingangs definierten Cyclohexenone I gefunden.

Diese neuen Cyclohexenonderivate haben eine gute herbizide Wirkung vorzugsweise gegen Arten aus der Familie der Gräser (Gramineen). Sie sind verträglich und somit selektiv in breitblättrigen Kulturen sowie in monokotylen Gewächsen, welche nicht zu den Gramineen zählen. Ferner sind Verbindungen darunter, welche sich auch in Gramineenkulturen wie Weizen, Gerste und Reis selektiv verhalten und gleichzeitig unerwünschte Gräser bekämpfen. Daneben zeigen die neuen Cyclohexenonderivate der Formel I wachstumsregulierende Wirkung gegen Arten aus der Familie der Gräser.

Die Herstellung der Verbindungen I erfolgt in vier Reaktionsschritten gemäß dem nachfolgenden Schema:

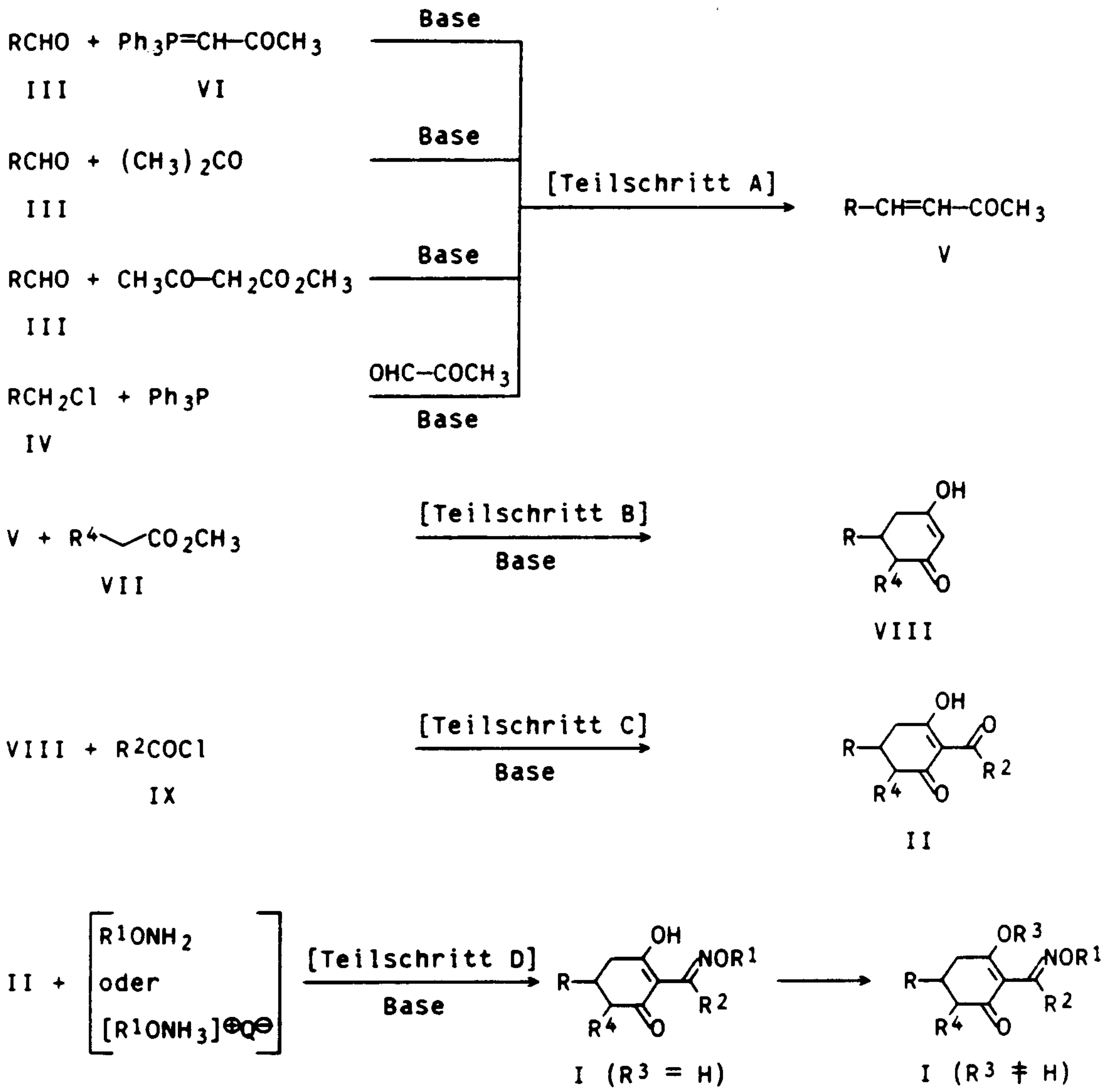


Die erforderlichen Zwischenprodukte sind durch eine Vielzahl von Methoden zugänglich, die in zahlreichen Patentveröffentlichungen, z.B. EP-A 125 094, EP-A 150 433 oder EP-A 238 021 beschrieben sind.

A) Teilschritt A umfaßt die Darstellung der Vinylketone der Formel V.

Alternativ sind z.B. folgende Verfahren möglich:

Ein Aldehyd der Formel III wird in Gegenwart einer Base mit Aceton oder mit einem Wittig-Reagenz der Formel VI oder mit Acetessigester in Gegenwart einer Base zum Vinylketon der Formel V umgesetzt.

Auch Halogenmethylverbindungen der Formel IV sind verwendbar, die zunächst in die Phosphoniumsalze übergeführt und anschließend mit Methylglyoxal zu den gewünschten Vinylketonen der Formel V umgesetzt werden.

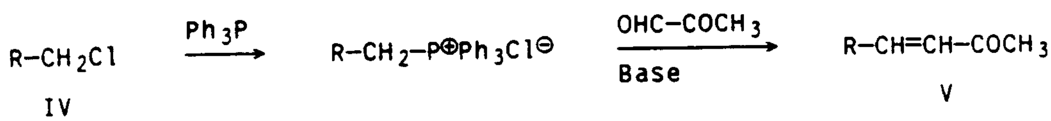


B) Teilschritt B betrifft die Synthese der 3-Hydroxy-2-cyclohexen-1-one der Formel VIII. Die Vinylketone der Formel V werden dazu beispielsweise mit einem Esterderivat der Formel VII in Gegenwart einer Base umgesetzt, wobei die gewünschten Verbindungen der Formel VIII entstehen.

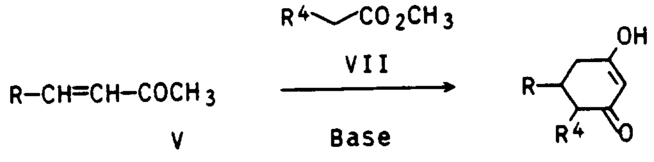


V VIII

C) Teilschritt C liefert die 2-Acyl-3-hydroxy-2-cyclohexen-1-one der Formel II. Die Derivate der Formel VIII werden dazu mit einem Säurechlorid der Formel IX in Gegenwart einer Base umgesetzt, wobei Oacylierte Derivate der Formel X entstehen, die anschließend in Gegenwart einer Lewis-Säure oder einer Base wie Imidazol, Pyridin oder 4-N,N-Dimethylaminopyridin in die C-acylierte Verbindung der Formel II umgelagert werden.

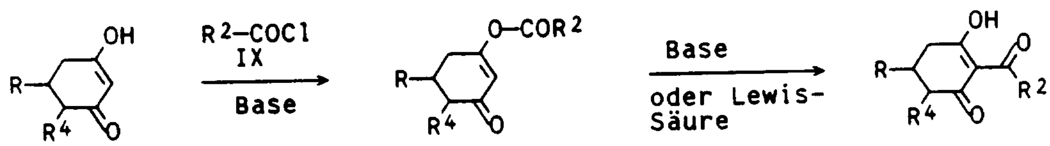


VIII X II

D) Teilschritt D betrifft die Darstellung der Verbindungen der Formel I, die aus den 2-Acyl-3-hydroxy-2-cyclohexen-1-onen II durch Umsetzung mit einem Alkoxyammonium-Salz der Formel $R^1ONH_2 \bullet HQ$, worin Q eine geeignete Abgangsgruppe wie Chlorid bedeutet, erhalten werden. Alternativ kann auch das freie Alkoxyamin $R^1ONH_2$ verwendet werden.

Verbindungen, die einen Substituenten $R^3 \neq$ Wasserstoff enthalten, sind aus Verbindungen der Formel I mit $R^3$ = H wie folgt in an sich bekannter Weise zugänglich.

Im Falle der Ether, Ester und Sulfone werden die Verbindungen der Formel I mit $R^3$ = H mit einem entsprechenden Alkylierungs-, Acyclierungs- oder Sulfonierungsmittel umgesetzt.

Geeignete Ausgangsmaterialien hierfür sind auch die Alkalimetallsalze der Verbindungen der Formel I, die durch Umsetzung mit Alkalimetallhydroxiden, -alkoholaten oder -hydriden gewonnen werden.

Bedeutet $R^3$ in Formel I ein sonstiges anorganisches oder organisches Kation, so sind diese Verbindungen aus den Alkalimetallsalzen mit übergangsmetallsalzen oder organischen Basen zugänglich. Beispiele hierfür sind Mangan-, Kupfer-, Zink- und Eisenchloride bzw. die entsprechenden Sulfate sowie Magnesium- und Calciummethylat bzw. -ethylat.

Die als Ausgangsmaterialien benötigten heterocyclischen Aldehyde III und Chlormethylverbindungen IV sind größtenteils neu, lassen sich aber nach allgemein üblichen Verfahren herstellen.

Die Darstellung der Isoxazole erfolgt z.B. nach den in DE-A 2 754 832 beschriebenen Bedingungen. Sie sind aus Aldoximen durch 1,3-dipolare Cycloaddition mit Propargylalkoholen oder -halogeniden zugänglich. Die 5-hydroxymethylsubstituierten Isoxazole lassen sich durch übliche Oxidationsverfahren in das 5-Formylderivat überführen. Die Regioisomeren, die in 3- bzw. 4-Position verknüpft sind, lassen sich u.a. nach Baraldi et al., J. Het. Chem. 19, 557 (1982) und Bertini et al., J. Chem. Soc. Perkin Trans I 1976, 570 herstellen.

Auf Synthesen für 1,3-Oxazole wird in "The Chemistry of Penicillines" eingegangen.

1,3-Thiazole, die in 4- oder 5-Position verknüpft sind, sind z.B. nach Verfahren entsprechend A. Beukö et al., Liebigs Ann. Chem. 717, 148-53 (1968) zugänglich.

Synthesen für 1,2,4-Oxadiazole werden beispielsweise in Chem. Ber. 17, 1685 (1889) und Tetrahedron Lett. 587-89 (1961) beschrieben.

Im Hinblick auf die bestimmungsgemäße Verwendung der Verbindungen I kommen als Substituenten bevorzugt die nachstehenden Reste in Betracht.

Alkylgruppen in der Bedeutung $R^1$ sind Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, wobei Ethyl und Propyl bevorzugt sind.

Alkenylgruppen in dieser Position sind 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-4-pentenyl, 1-Methyl-4-pentenyl, 2-Methyl-4-pentenyl, 3-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 2-Ethyl-2-butenyl, 1-Ethyl-3-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl und 2-Butenyl.

Als Reste $R^1$ sind von den Alkinylgruppen 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 4-Methyl-2-pentinyl, 1-Methyl-3-pentinyl, 2-Methyl-3-pentinyl, 1-Methyl-4-pentinyl, 2-Methyl-4-pentinyl, 3-Methyl-4-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, insbesondere 2-Propinyl und 2-Butinyl bevorzugt.

Diese Alkyl-, Alkenyl- und Alkinylgruppen können durch ein bis drei der folgenden Gruppen substituiert sein:
Halogenatome wie Brom und Jod, vorzugsweise jedoch ein bis drei Fluor- und/oder Chloratome; besonders bevorzugt ist (E)-3-Chlorprop-2-enyl;
Alkoxygruppen wie Methoxy, Ethoxy, Propyloxy, 1-Methylethyloxy, Butyloxy, 1-Methylpropyloxy, 2-Methylpropyloxy und 1,1-Dimethylethyloxy, insbesondere ein bis zwei Methoxy- und Ethoxygruppen;
Alkylthiogruppen wie Methylthio, Ethylthio, Propylthio, 1-Methylethylthio, Butylthio, 1-Methylpropylthio, 2-Methylpropylthio und 1,1-Dimethylethylthio, wobei ein bis zwei Ethylthiogruppen bevorzugt sind, und/oder ein Phenylring, welcher neben den oben genannten Halogenatomen, Alkylgruppen, Alkoxygruppen und Alkylthiogruppen auch durch Cyanogruppen und Halogenalkylgruppen wie Fluormethyl, Difluormethyl, Trifluormethyl, Chlormethyl, Dichlormethyl, Trichlormethyl, Difluorchlormethyl, 1-Fluorethyl, 2-Fluorethyl, 1,1-Difluorethyl, 1,1,2-Trifluorethyl, 1,2,2-Trifluorethyl und 2,2,2-Trifluorethyl tragen kann, insbesondere ist der Phenylring unsubstituiert oder in 4-Position durch Fluor, Chlor, Methyl, Trifluormethyl, 1,1-Dimethylethyl und/oder in 2-Position durch Chlor oder Methoxy substituiert.

$R^1$ kann daneben auch die Bedeutung Phenyl, Thenyl oder Benzyl haben, wobei die aromatischen Kerne neben den oben genannten Halogenatomen, Alkoxygruppen, Alkylthiogruppen und Halogenalkylgruppen auch durch Nitro-, Cyano- und/oder Alkylgruppen, wie Methyl, Ethyl, Propyl, 1-Methylethyl, Butyl, 1-Methylpropyl, 2-Methylpropyl und 1,1-Dimethylethyl substituiert sein können. Besonders bevorzugt ist der 5-Chlorthenylrest.

Der Rest $R^2$ hat im allgemeinen und im besonderen bezüglich der Alkyl-, Alkenyl- und Alkinylgruppen, des Phenylrestes sowie dessen Substituenten die gleiche Bedeutung wie $R^1$.

Als Reste $R^3$ kommen neben den bereits aufgeführten Alkyl-, Alkenyl- und Alkinylgruppen auch Reste von gesättigten und ungesättigten Carbonsäuren, wie Acetyl, Propionyl, Butyryl und Pivaloyl, insbesondere Acetyl und Butyryl;

sowie Alkylsulfonyl-, -silyl-, -phosphonyl- oder -thiophosphonylgruppen, wobei die Alkylreste vorzugsweise Methyl-, Ethyl-, 1-Methylethyl- und 1,1-Dimethylethylgruppen sind,
und der Benzoyl- sowie der Phenylsulfonylrest in Betracht. Die letztgenannten aromatischen Reste können dabei bis zu dreifach substituiert sein. Die möglichen Substituenten entsprechen den unter $R^1$ aufgeführten Untersubstituenten.

Als Reste $R^4$ kommen neben Wasserstoff und Cyanogruppen die bereits bei $R^1$ erwähnten Halogenatome und Alkylgruppen sowie Alkoxycarbonylgruppen, wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl und 1,1-Dimethylethoxycarbonyl, besonders jedoch Methoxycarbonyl und Ethoxycarbonyl in Betracht.

Bevorzugte Reste $R^4$ sind Wasserstoff, Cyano und Methoxycarbonyl.

Unter A ist eine bis zu viergliedrige Alkylen-, Alkylenoxyalkylen- oder Alkylenthioalkylenkette zu verstehen. Beispiele dafür sind Methylen-, Ethylen-, Propylen-, Butylen-, Methylenoxymethylen-, Methylenoxyethylen-, Methylenthiomethylen-, Methylenthioethylen-, Propylenoxy-, Propylenthio-, Ethylenoxy-, Ethylenthio-, Methylenoxy-, Methylenthio-, Oxi- und Thiobrücken.

Vorzugsweise enthalten die Verbindungen der Formel I keine oder eine 1- oder 2-gliedrige Brücke A.

Unter den 5-gliedrigen Heterocyclen als Rest B sind Ringe, wie Dihydrofuranyl, Dihydrothienyl, Pyrrolinyl, Pyrazolinyl, Imidazolinyl, Isoxazolinyl, Oxazolinyl, Isothiazolinyl, Thiazolinyl, Furanyl, Thienyl,

Pyrrolyl, Pyrazolyl, Isoxazolyl, Oxazolyl, Isothiazolyl, Thiazolyl, Oxadiazolyl und Thiadiazolyl zu verstehen.

Besonders bevorzugte Verbindungen der Formel I sind solche in denen ein 4,5-Dihydroisoxazolylring über seine 4- oder 5-Stellung mit einer der obengenannten Brücken an das Cyclohexenonsystem gebunden ist sowie solche in denen einer der aufgezählten heteroaromatischen Ringe direkt an den Cyclohexenonring gebunden ist.

In der Bedeutung X kommen folgende Gruppen in Betracht:

z.B. Pyrrolyl, Pyrazolyl, Imidazolyl, Triazolyl, Pyridyl, Pyrimidyl, Pyrazinyl, Pyridazinyl, Chinolinyl oder Isochinolinyl, Furanyl, Thienyl, Isoxazolyl, Oxazolyl, Oxathiazolyl, Isothiazolyl und Thiazolyl; bevorzugt sind 2-Pyridyl und 2-Furyl.

Die heteroaromatischen Ringe können dabei bis zu drei Substituenten tragen. Neben Cyano- und Nitrogruppen kommen hierfür die bereits genannten Halogenatome, Alkylgruppen, Halogenalkylgruppen, Alkoxygruppen und Alkylthiogruppen in Betracht.

Unter Yn sind ein bis drei gleiche oder verschiedene Reste der bereits erwähnten Alkyl-, Alkenyl-oder Alkinylgruppen sowie Wasserstoff, Halogen, Cyano und Nitro zu verstehen. Zusätzlich kann Alkenyl hier insbesondere Vinyl und Alkinyl insbesondere Ethinyl bedeuten.

Die Erfindung umfaßt im einzelnen vorzugsweise die folgenden Strukturen:

a) Pyrrole der Formel

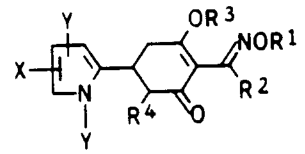

I.01

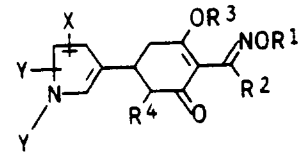

I.02

b) Furane und Thiophene der Formel

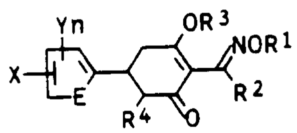

I.03 E=O
I.04 E=S

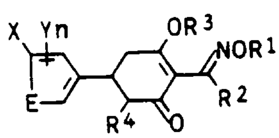

I.05 E=O
I.06 E=S

c) Pyrazole der Formel

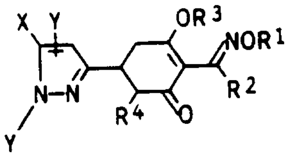

I.07

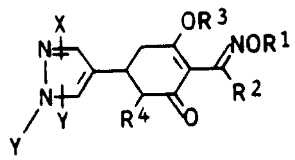

I.08

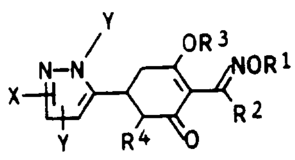

I.09

d) Isoxazole und Isothiazole der Formel

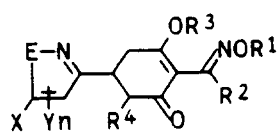

I.10 E=O
I.11 E=S

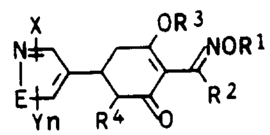

I.12 E=O
I.13 E=S

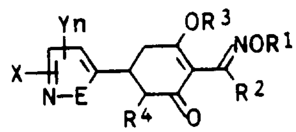

I.14 E=O
I.15 E=S

e) Oxazole und Thiazole der Formel

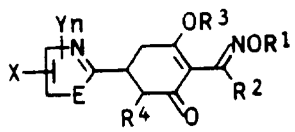

I.16 E=O
I.17 E=S

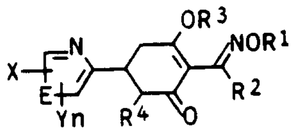

I.18 E=O
I.19 E=S

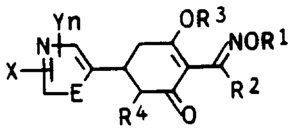

I.20 E=O
I.21 E=S

f) 1,2,3-Oxadiazole und 1,2,3-Thiadiazole der Formel

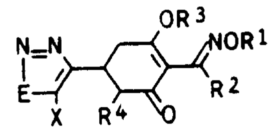

I.22 E=O
I.23 E=S

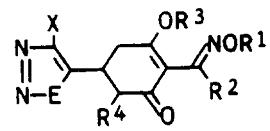

I.24 E=O
I.25 E=S

g) 1,2,4-Oxadiazole und 1,2,4-Thiadiazole der Formel

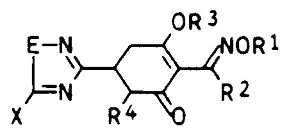

I.26 E=O
I.27 E=S

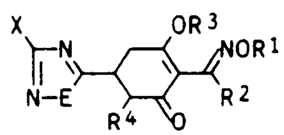

I.28 E=O
I.29 E=S

h) 1,3,4-Oxadiazole und 1,3,4-Thiadiazole der Formel

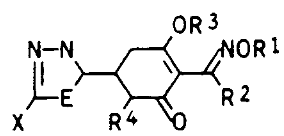

I.30 E=O
I.31 E=S

i) 4,5-Dihydroisoxazoline der Formel

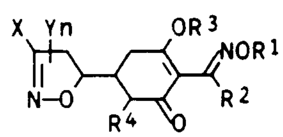

I.32

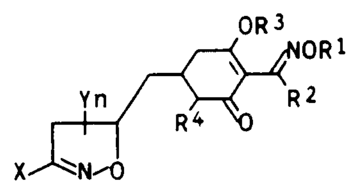

I.33

Die Cyclohexenonderivate I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Cyclohexenone I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanole, sowie von Fettalkoholglykolethern, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,1 und 95 Gew.%, vorzugsweise zwischen 0,5 und 90 Gew.%, Wirkstoff.

Die Applikation der herbiziden Mittel kann im Vorauflaufverfahren oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,01 bis 3 kg/ha, vorzugsweise 0,05 bis 1,0 kg/ha.

In Anbetracht des erfaßbaren Wirkungsspektrums zur Unkrautbekämpfung, der Verträglichkeit für Kulturpflanzen oder der erwünschten Beeinflussung des Wachstums derselben sowie angesichts der Vielfalt der Applikationsmethoden können die erfindungsgemäßen Verbindungen in einer großen Zahl von Kulturpflanzen eingesetzt werden. In Betracht kommen beispielsweise folgende Kulturen:

Kulturenliste

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |

| Botanischer Name | Deutscher Name |
|---|---|
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |

| Botanischer Name | Deutscher Name |
|---|---|
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Cyclohexenonderivate der Formel I sowohl untereinander als auch mit Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren, Cyclohexenone, (Hetero)-aryloxyphenoxypropionsäuren, deren Salze, Ester und Amide sowie andere in Betracht.

Außerdem kann es von Nutzen sein, die Cyclohexenonderivate der Formel I bzw. sie enthaltende herbizide Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

In den folgenden Synthesevorschriften werden die einzelnen Teilschritte der Synthese anhand konkreter Beispiele erläutert.

1. 5-Chlormethyl-3-(2-pyridyl)-isoxazol

Eine Mischung aus 122 g 2-Pyridylaldoxim, 1,5 l Wasser, 500 ml Dichlormethan, 15 g Natriumdihydrogenphosphatdihydrat, 18 g Natriumhydrogenphosphatdihydrat und 150 g Propargylchlorid wurde bei 20-25 °C mit 774 g einer 14%igen Natriumhypochloritlösung versetzt. Es wurde bei 25 °C 10 Stunden gerührt, die Phasen getrennt und die wäßrige Phase zweimal mit je 150 ml Dichlormethan extrahiert. Die

vereinigten organischen Phasen wurden über Natriumsulfat getrocknet und eingeengt. Man erhielt 170 g eines braunen Feststoffs. Das Produkt wird ohne weitere Reinigung umgesetzt.

2. Darstellung der Vinylketone V

a) 4-[3-(2-Pyridyl)-isoxazol-5-yl]-3-buten-2-on

100 g 5-Chlormethyl-3-(2-pyridyl)-isoxazol und 175 g Triphenylphosphan wurden in 300 ml Chloroform gelöst und 2 Tage bei Rückflußtemperatur gerührt. Nach dem Abkühlen und Einengen des Reaktionsansatzes wurde der Rückstand mit tert.-Butyl-methylether verrührt, der braune Feststoff abgetrennt und getrocknet. Es wurden 210 g Phosphoniumsalz erhalten, die mit 113 g einer 40%-igen Methylglyoxallösung in Wasser sowie je 250 ml Wasser und Dichlormethan versetzt wurden. Bei 25 °C wurden in kleinen Portionen 46 g Natriumhydrogencarbonat hinzugesetzt und anschließend 1 Stunde gerührt, die organische Phase abgetrennt und die wäßrige Phase zweimal mit Dichlormethan extrahiert. Die vereinigten organischen Phasen wurden eingeengt und mit tert.-Butyl-methylether verrührt. Man erhielt 100 g eines hellbraunen Feststoffes, welcher ohne weitere Reinigung umgesetzt wird.

b) 4-[3-(2-Tetrahydropyranyl)-isoxazol-5-yl]-3-buten-2-on (fällt nicht mehr unter die Erfindung)

47 g 1-Triphenylphosphoranyliden-2-propanon wurden in 50 ml Dichlormethan gelöst und mit 27 g 5-Formyl-3-(2-tetrahydropyranyl)-isoxazol versetzt. Die Reaktionsmischung wurde 1 d bei Raumtemperatur gerührt, dann eingeengt und in Methyl-tert.-butylether verrührt. Nach Abtrennung des Triphenylphosphinoxids bleiben 43 g eines gelben Öls zurück.

3. Darstellung der 3-Hydroxy-2-cyclohexen-1-one VIII

3-Hydroxy-5-[-3-(3-tetrahydrofuryl)isoxazol-5-yl]-2-cyclohexen-1-on (fällt nicht mehr unter die Erfindung)

10,6 g Dimethylmalonat wurden in 300 ml Methanol vorgelegt und bei Raumtemperatur mit 14,4 g einer 30%igen Natriummethanolatlösung in Methanol versetzt. Anschließend wurden 16,0 g 4-[3-(Tetrahydrofuryl)isoxazol-5-yl]-3-buten-2-on hinzugefügt und 1 Tag bei 25 °C gerührt. Der nach dem Entfernen des Lösungsmittels verbliebene Rückstand wurde in 10%iger Natronlauge aufgenommen und 24 Stunden bei 25 °C gerührt. Die Natronlaugephase wurde mit Methyl-tert.-butylether gewaschen, anschließend mit konz. Salzsäure bis pH 2 angesäuert und 2 Stunden auf 85 °C erwärmt. Der gebildete Feststoff wurde abfiltriert, mit Wasser gewaschen und getrocknet. Man erhielt 12,3 g eines beigefarbenen Feststoffs, Schmp. 142-144 °C.

4. Darstellung der 2-Acyl-3-hydroxy-2-cyclohexen-1-one II

2-Butyryl-3-hydroxy-5-[3-(tetrahydrofuryl)isoxazol-5-yl]-2-cyclohexen-1-on (fällt nicht mehr unter die Erfindung)

12,0 g des Diketons aus 3a) wurden in 300 ml Tetrahydrofuran gelöst, mit 5,4 g Triethylamin und anschließend mit 5,6 g Buttersäurechlorid versetzt. Die Reaktionsmischung rührte 24 Stunden bei 25 °C, wurde dann mit Wasser versetzt und mit Dichlormethan extrahiert. Die organische Phase wurde einmal mit Wasser gewaschen, getrocknet und eingeengt. Es blieben 15,2 g eines orangefarbenen Öls zurück, das in 150 ml Dichlormethan aufgenommen und mit 3,0 g 4-N,N-Dimethylaminopyridin versetzt wurde. Nach 3 Tagen wurde das Gemisch in 10 %ige Salzsäure eingerührt. Die organische Phase wurde abgetrennt, einmal mit Wasser gewaschen, getrocknet und eingeengt. Man erhielt 14,1 g eines gelben Feststoffes, Schmp. 102-104 °C.

5. Darstellung der Cyclohexenonoximetherderivate der Formel I

2-(1-Ethoxyiminobutyl)-3-hydroxy-5-[3-(3-tetrahydrofuryl)-isoxazol-5-yl]-2-cyclohexen-1-on (fällt nicht mehr unter die Verbindung)

Eine Mischung aus 3,0 g der Acylverbindung gemäß 4a), 0,9 g Natriumhydrogencarbonat, 1,1 g Ethoxyaminhydrochlorid und 100 ml Methanol wurden 24 Stunden bei 25 °C gerührt, anschließend eingeengt, in Essigsäureethylester aufgenommen und chromatographisch gereinigt.

Man erhielt 2,5 g eines gelben Feststoffs, Schmp. 58-60 °C .

In entsprechender Weise können die in den folgenden Tabellen aufgeführten Verbindungen der Formel I hergestellt werden, die durch ihren Schmelzpunkt (Schmp.) charakterisiert sind. In Tabelle 7 sind diejenigen Verbindungen aufgelistet, die nicht als Feststoff isoliert werden konnten. Verbindungen, bei denen die physikalischen Daten fehlen, können in analoger Weise erhalten werden.

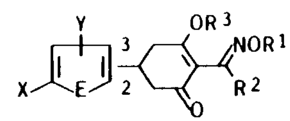

Tabelle 1

| Verb. Nr. | E | Bindung in Position | X | Y | $R^3$ | $R^2$ | $R^1$ | Schmp./°C |
|---|---|---|---|---|---|---|---|---|
| 1.1 | S | 2 | 2-Thienyl | H | H | n-Propyl | Ethyl | 51-52 |
| 1.2 | S | 2 | 2-Thienyl | H | H | n-Propyl | Allyl | 53-55 |
| 1.3 | S | 2 | 2-Thienyl | H | H | n-Propyl | (E)-2-Butenyl | 56-57 |
| 1.4 | S | 2 | 2-Thienyl | H | H | n-Propyl | (E)-3-Chlor-2-propenyl | 65-67 |
| 1.5 | S | 2 | 2-Thienyl | H | H | Ethyl | Ethyl | |
| 1.6 | S | 2 | 2-Thienyl | H | H | Ethyl | Allyl | 47-48 |
| 1.7 | S | 2 | 2-Thienyl | H | H | Ethyl | (E)-2-Butenyl | 75-77 |
| 1.8 | S | 2 | 2-Thienyl | H | H | Ethyl | (E)-3-Chlor-2-propenyl | 58-61 |
| 1.9 | S | 2 | 5-Chlor-2-thienyl | H | H | n-Propyl | Ethyl | |
| 1.10 | S | 2 | 5-Chlor-2-thienyl | H | H | n-Propyl | (E)-2-Butenyl | |
| 1.11 | S | 2 | 5-Chlor-2-thienyl | H | H | n-Propyl | 2-Butinyl | |

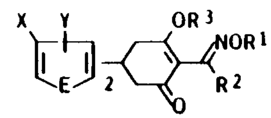

Tabelle 2

| Verb. Nr. | E | Bindung in Position | X | Y | $R^3$ | $R^2$ | $R^1$ | Schmp./°C |
|---|---|---|---|---|---|---|---|---|
| 2.1 | S | 2 | 1-Imidazolyl | H | H | Ethyl | Ethyl | |
| 2.2 | S | 2 | 1-Imidazolyl | H | H | Ethyl | Allyl | |
| 2.3 | S | 2 | 1,3-Thiazol-2-yl | 5-Methyl | H | n-Propyl | 2-Fluorethyl | |
| 2.4 | S | 2 | 1,3-Thiazol-2-yl | 5-Methyl | H | n-Propyl | 2-Propinyl | |
| 2.5 | S | 2 | 1,3-Thiazol-2-yl | 5-Methyl | H | n-Propyl | (E)-2-Butenyl | |

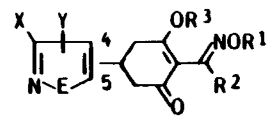

Tabelle 3

| Verb. Nr. | E | Bindung in Position | X | Y | $R^3$ | $R^2$ | $R^1$ | Schmp. °C |
|---|---|---|---|---|---|---|---|---|
| 3.1 | O | 5 | 2-Furyl | H | H | Ethyl | Ethyl | 120-121 |
| 3.2 | O | 5 | 2-Furyl | H | H | Ethyl | Allyl | 83 |
| 3.3 | O | 5 | 2-Furyl | H | H | Ethyl | (E)-2-Butenyl | 109-110 |
| 3.4 | O | 5 | 2-Furyl | H | H | Ethyl | (E)-3-Chlor-2-propenyl | 108-109 |
| 3.5 | O | 5 | 2-Furyl | H | H | n-Propyl | Ethyl | Öl |

Tabelle 3 (Fortsetzung)

| Verb. Nr. | E | Bindung in Position | X | Y | $R^3$ | $R^2$ | $R^1$ | Schmp./°C |
|---|---|---|---|---|---|---|---|---|
| 3.6 | 0 | 5 | 2-Furyl | H | H | n-Propyl | Allyl | 56-57 |
| 3.7 | 0 | 5 | 2-Furyl | H | H | n-Propyl | (E)-2-Butenyl | 73-74 |
| 3.8 | 0 | 5 | 2-Furyl | H | H | n-Propyl | (E)-3-Chlor-2-propenyl | 100-101 |
| 3.9 | 0 | 5 | 3-Pyridyl | H | H | n-Propyl | Ethyl | 117-119 |
| 3.10 | 0 | 5 | 3-Pyridyl | H | H | n-Propyl | (E)-3-Chlor-2-propenyl | |
| 3.11 | 0 | 5 | 3-Pyridyl | H | H | n-Propyl | (E)-4-(3-Trifluormethyl-phenyl)-2-butenyl | |
| 3.12 | 0 | 5 | 3-Pyridyl | H | H | n-Propyl | (E)-2-Butenyl | 108-110 |
| 3.13 | 0 | 5 | 2-Pyridyl | H | H | Ethyl | Ethyl | 73 |
| 3.14 | 0 | 5 | 2-Pyridyl | H | H | Ethyl | (E)-2-Butenyl | 93 |
| 3.15 | 0 | 5 | 2-Pyridyl | H | H | Ethyl | (E)-4-(4-Chlorphenyl)-2-butenyl | |
| 3.16 | 0 | 5 | 2-Pyridyl | H | H | n-Propyl | Ethyl | 75 |
| 3.17 | 0 | 5 | 2-Pyridyl | H | H | n-Propyl | (E)-3-Chlor-2-propenyl | 84 |
| 3.18 | 0 | 5 | 2-Pyridyl | H | H | n-Propyl | (E)-2-Butenyl | 63-64 |
| 3.19 | 0 | 5 | 2-Pyridyl | H | H | n-Propyl | Allyl | Öl |
| 3.20 | 0 | 5 | 2-Pyridyl | H | H | Ethyl | Allyl | 75-76 |
| 3.21 | 0 | 5 | 2-Pyridyl | H | H | Ethyl | (E)-3-Chlor-2-propenyl | 112 |

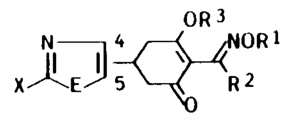

Tabelle 4

| Verb. Nr. | E | X | Bindungsstelle | $R^3$ | $R^2$ | $R^1$ | Schmp./°C |
|---|---|---|---|---|---|---|---|
| 4.1 | S | 4-Pyridyl | 4 | H | Ethyl | 2-Chlorethyl | |
| 4.2 | S | 4-Pyridyl | 4 | H | Ethyl | (E)-3-Chlor-2-propenyl | |
| 4.3 | S | 4-Pyridyl | 4 | H | Ethyl | 5-Chlor-2-thenyl | |
| 4.4 | S | 4-Pyridyl | 4 | H | n-Propyl | Ethyl | |

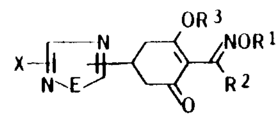

Tabelle 5

| Verb. Nr. | E | Bindung in Position | X | $R^3$ | $R^2$ | $R^1$ | Schmp./°C |
|---|---|---|---|---|---|---|---|
| 5.1 | O | 5 | 2-Furyl | H | Ethyl | 5-Chlor-2-thenyl | |
| 5.2 | O | 5 | 2-Furyl | H | Ethyl | (E)-4-Phenyl-2-butenyl | |
| 5.3 | O | 5 | 2-Pyridyl | H | n-Propyl | 2-Fluorethyl | |
| 5.4 | O | 5 | 2-Pyridyl | H | n-Propyl | Ethyl | |

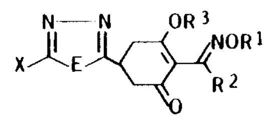

Tabelle 6

| Verb. Nr. | E | X | $R^3$ | $R^2$ | $R^1$ | Schmp./°C |
|---|---|---|---|---|---|---|
| 6.1 | S | 2-Furyl | H | Ethyl | Ethyl | |
| 6.2 | S | 2-Furyl | H | Ethyl | (E)-2-Butenyl | |
| 6.3 | S | 2-Furyl | Li | Ethyl | 5-Chlor-2-thenyl | |

Die $^1H$-NMR-Spektren wurden in Deuteriochloroform oder Hexadeuteriodimethylsulfoxid als Lösungsmittel mit Tetramethylsilan als internem Standard aufgenommen. Die chemischen Verschiebungen werden in (ppm) registriert. Die Multiplizitäten werden wie folgt angegeben: s = Singulett, d = Dublett, t = Triplett, q = Quartett, m = Multiplett.

Tabelle 7

$^1H$-NMR-Daten

| Verb. | chem. Verschiebung |
|---|---|
| 1.5 | 1.16(t,3H), 1.34(t,3H), 3.59(m,1H), 4.12(q,2H) |
| 3.5 | 1.0 (t,3H), 1.35(t,3H), 2.97(t,2H), 4.14(q,2H) 6,35(2,1H) |

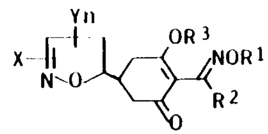

Tabelle 8

| Verbindung | X | Yn | R3 | R2 | R1 | Schmp. °C |
|---|---|---|---|---|---|---|
| 8.1 | 3-(2-Pyridyl) | 5-Methyl | H | Ethyl | Ethyl | |
| 8.2 | 3-(2-Pyridyl) | 5-Methyl | H | Ethyl | (E)-2-Butenyl | |
| 8.3 | 3-(2-Pyridyl) | 5-Methyl | H | Ethyl | (E)-3-Chlor-2-propenyl | |
| 8.4 | 3-(2-Pyridyl) | 5-Methyl | H | n-Propyl | Ethyl | |
| 8.5 | 3-(2-Pyridyl) | 5-Methyl | H | n-Propyl | Allyl | |
| 8.6 | 3-(2-Pyridyl) | 5-Methyl | H | n-Propyl | 2-Propinyl | |
| 8.7 | 3-(2-Furyl) | 5-i-Propyl | H | n-Propyl | Ethyl | |
| 8.8 | 3-(2-Furyl) | 5-i-Propyl | H | n-Propyl | 5-Chlor-2-thenyl | |
| 8.9 | 3-(2-Furyl) | 5-i-Propyl | H | n-Propyl | (E)-4-(4-Chlor-phenyl)-2-butenyl | |

Die in den Gewächshausversuchen verwendeten Pflanzen setzten sich aus folgenden Arten zusammen:

| Botanischer Name | Deutscher Name |
|---|---|
| Avena fatua | Flughafer |
| Digitaria sanguinalis | Blutfingerhirse |
| Echinochloa crus-galli | Hühnerhirse |
| Lolium multiflorum | Ital. Raygrass |
| Medicago sativa | Luzerne |
| Setaria viridis | Grüne Borstenhirse |
| Sinapis alba | Weißer Senf |
| Triticum aestivum | Winterweizen |
| Zea mays | Mais |

**Patentansprüche**
**Patentansprüche für folgende Vertragsstaaten : BE, CH, DE, FR, GB, IT, LI, NL**

**1.** Cyclohexenonderivate der allgemeinen Formel I

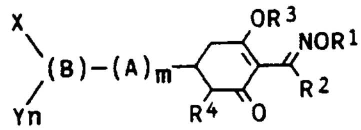
I

in der die Substituenten folgende Bedeutung haben:

$R^1$ eine $C_1$-$C_6$-Alkylgruppe, eine $C_3$-$C_6$-Alkenylgruppe oder eine $C_3$-$C_6$-Alkinylgruppe, wobei diese Gruppen ein bis drei der folgenden Reste tragen können: Halogen, $C_1$-$C_4$-Alkoxy, $C_1$-$C_4$-Alkylthio und/oder Phenyl, wobei die Phenylgruppe ihrerseits bis zu dreifach durch Halogen, $C_1$-$C_4$-Alkyl, $C_1$-$C_4$-Alkoxy, $C_1$-$C_4$-Alkylthio, $C_1$-$C_4$-Halogenalkyl und/oder Cyano substituiert sein kann;
eine Phenylgruppe, eine Benzylgruppe oder eine Thenylgruppe, wobei die aromatischen Kerne ein bis drei der folgenden Reste tragen können: Halogen, $C_1$-$C_4$-Alkyl, $C_1$-$C_4$-Alkoxy, $C_1$-$C_4$-Alkylthio, $C_1$-$C_4$-Halogenalkyl, Nitro und/oder Cyano;

$R^2$ eine $C_1$-$C_6$-Alkylgruppe, eine $C_3$-$C_6$-Alkenylgruppe, eine $C_3$-$C_6$-Alkinylgruppe oder eine Phenylgruppe, wobei diese Gruppen ein bis drei der folgenden Reste tragen können: Halogen, $C_1$-$C_4$-Alkoxy, $C_1$-$C_4$-Alkylthio und/oder Cyano;

$R^3$ Wasserstoff, eine $C_1$-$C_6$-Alkylgruppe, eine $C_1$-$C_{10}$-Alkylcarbonylgruppe, eine $C_1$-$C_6$-Alkylsulfonylgruppe, eine $C_1$-$C_4$-Trialkylsilylgruppe, eine $C_1$-$C_4$-Dialkylphosphonylgruppe, eine $C_1$-$C_4$-Dialkylthiophosphonylgruppe, eine $C_3$-$C_6$-Alkenylgruppe, eine $C_3$-$C_6$-Alkenylcarbonylgruppe, eine $C_3$-$C_6$-Alkinylgruppe oder
eine Benzoyl- oder Phenylsulfonylgruppe, die ein- bis dreifach durch Halogen, Nitro, Cyano, $C_1$-$C_4$-Alkyl, $C_1$-$C_4$-Halogenalkyl, $C_1$-$C_4$-Alkoxy und/oder $C_1$-$C_4$-Alkylthio substituiert sein können;

$R^4$ Wasserstoff, eine Cyanogruppe, ein Halogenatom, eine $C_1$-$C_6$-Alkylgruppe oder eine $C_1$-$C_6$-Alkoxycarbonylgruppe;

A eine $C_1$-$C_4$-Alkylenkette, in der eine Methylengruppe durch ein Sauerstoff- oder Schwefelatom ersetzt sein kann und die ein bis zwei der folgenden Substituenten tragen kann: $C_1$-$C_4$-Alkyl, $C_1$-$C_4$-Alkoxy, $C_1$-$C_4$-Alkylthio und/oder Halogen;

m 0 oder 1

B ein 5-gliedriger einfach oder doppelt ungesättigter Heterocyclus mit bis zu 2 Stickstoffatomen und/oder bis zu einem Sauerstoff- oder Schwefelatom;

X ein 5- 6-gliedriger Heteroaromat, wobei diese Ringe ein bis drei der folgenden Gruppen tragen können: $C_1$-$C_4$-Alkyl, $C_1$-$C_4$-Alkoxy, $C_1$-$C_4$-Alkylthio, $C_1$-$C_4$-Halogenalkyl, Halogen, Cyano und/oder Nitro;

Y Wasserstoff, ein Halogenatom, eine Cyanogruppe, eine Nitrogruppe; eine

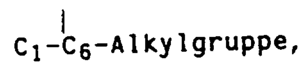

eine $C_2$-$C_6$-Alkenylgruppe oder eine $C_2$-$C_6$-Alkinylgruppe, wobei diese Gruppen ein bis drei der folgenden Reste tragen können: Phenyl, Halogen, $C_1$-$C_4$-Alkoxy und/oder $C_1$-$C_4$-Alkylthio, eine $C_1$-$C_4$-Alkoxygruppe, eine $C_1$-$C_4$-Alkylthiogruppe, eine $C_3$-$C_6$-Alkenyloxygruppe oder eine $C_3$-$C_6$-Alkinyloxygruppe;

n 1, 2 oder 3 ;

sowie deren umweltverträglichen Salze.

**2.** Verfahren zur Herstellung der Verbindungen I gemäß Anspruch 1, dadurch gekennzeichnet, daß man eine Verbindung der Formel II

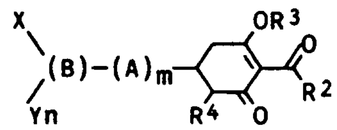

II

in an sich bekannter Weise

a) mit einem Hydroxylaminderivat der Formel $[R^1ONH_3]^{\oplus}Q^{\ominus}$, in der $Q^{\ominus}$ ein beliebiges Anion bedeutet, in einem inerten Lösungsmittel bei einer Temperatur zwischen 0 und 80 ° C, in Gegenwart oder Abwesenheit einer Hilfsbase umsetzt oder

b) in einem inerten Lösungsmittel mit einem Hydroxylamin der Formel $R^1ONH_2$ bei Temperaturen zwischen 0 und 90 ° C umsetzt.

**3.** Herbizides Mittel, enthaltend ein Cyclohexenonderivat der Formel I gemäß Anspruch 1 und inerte Zusatzstoffe.

**4.** Herbizides Mittel nach Anspruch 3, enthaltend weitere wirksame Bestandteile.

**5.** Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Cyclohexenonderivats der Formel I gemäß Anspruch 1 behandelt.

**6.** Verwendung der Cyclohexenonderivate der Formel I gemäß Anspruch 1 als Herbizide.

**Patentansprüche für folgenden Vertragsstaat : ES**

**1.** Verfahren zur Herstellung von Cyclohexenonderivaten der allgemeinen Formel I

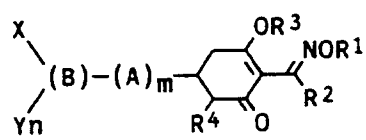

I

in der die Substituenten folgende Bedeutung haben:

$R^1$ eine $C_1$-$C_6$-Alkylgruppe$_1$ eine $C_3$-$C_6$-Alkenylgruppe oder eine $C_3$-$C_6$-Alkinylgruppe, wobei diese Gruppen ein bis drei der folgenden Reste tragen können: Halogen, $C_1$-$C_4$-Alkoxy, $C_1$-$C_4$-Alkylthio und/oder Phenyl, wobei die Phenylgruppe ihrerseits bis zu dreifach durch Halogen, $C_1$-$C_4$-Alkyl, $C_1$-$C_4$-Alkoxy, $C_1$-$C_4$-Alkylthio, $C_1$-$C_4$-Halogenalkyl und/oder Cyano substituiert sein kann;

eine Phenylgruppe, eine Benzylgruppe oder eine Thenylgruppe, wobei die aromatischen Kerne ein bis drei der folgenden Reste tragen können: Halogen, $C_1$-$C_4$-Alkyl, $C_1$-$C_4$-Alkoxy, $C_1$-$C_4$-Alkylthio, $C_1$-$C_4$-Halogenalkyl, Nitro und/oder Cyano;

$R^2$ eine $C_1$-$C_6$-Alkylgruppe, eine $C_3$-$C_6$-Alkenylgruppe, eine $C_3$-$C_6$-Alkinylgruppe oder eine Phenylgruppe, wobei diese Gruppen ein bis drei der folgenden Reste tragen können: Halogen, $C_1$-$C_4$-Alkoxy, $C_1$-$C_4$-Alkylthio und/oder Cyano;

$R^3$ Wasserstoff, eine $C_1$-$C_6$-Alkylgruppe, eine $C_1$-$C_{10}$-Alkylcarbonylgruppe, eine $C_1$-$C_6$-Alkylsulfonylgruppe, eine $C_1$-$C_4$-Trialkylsilylgruppe, eine $C_1$-$C_4$-Dialkylphosphonylgruppe, eine $C_1$-$C_4$-Dialkylthiophosphonylgruppe, eine $C_3$-$C_6$-Alkenylgruppe, eine $C_3$-$C_6$-Alkenylcarbonylgruppe, eine $C_3$-$C_6$-Alkinylgruppe oder
eine Benzoyl- oder Phenylsulfonylgruppe, die ein- bis dreifach durch Halogen, Nitro, Cyano, $C_1$-$C_4$-Alkyl, $C_1$-$C_4$-Halogenalkyl, $C_1$-$C_4$-Alkoxy und/oder $C_1$-$C_4$-Alkylthio substituiert sein können;

$R^4$ Wasserstoff, eine Cyanogruppe, ein Halogenatom, eine $C_1$-$C_6$-Alkylgruppe oder eine $C_1$-$C_6$-Alkoxycarbonylgruppe;

A eine $C_1$-$C_4$-Alkylenkette, in der eine Methylengruppe durch ein Sauerstoff- oder Schwefelatom ersetzt sein kann und die ein bis zwei der folgenden Substituenten tragen kann: $C_1$–$C_4$-Alkyl, $C_1$-$C_4$-Alkoxy, $C_1$-$C_4$-Alkylthio und/oder Halogen;

m 0 oder 1

B ein 5-gliedriger einfach oder doppelt ungesättigter Heterocyclus mit bis zu 2 Stickstoffatomen und/oder bis zu einem Sauerstoff- oder Schwefelatom;

X ein 5- 6-gliedriger Heteroaromat, wobei diese Ringe ein bis drei der folgenden Gruppen tragen können: $C_1$-$C_4$-Alkyl, $C_1$-$C_4$-Alkoxy, $C_1$-$C_4$-Alkylthio, $C_1$-$C_4$-Halogenalkyl, Halogen, Cyano und/oder Nitro;

Y Wasserstoff, ein Halogenatom, eine Cyanogruppe, eine Nitrogruppe; eine

$C_1$-$C_6$-Alkylgruppe,

eine $C_2$-$C_6$-Alkenylgruppe oder eine $C_2$-$C_6$-Alkinylgruppe, wobei diese Gruppen ein bis drei der folgenden Reste tragen können: Phenyl, Halogen, $C_1$-$C_4$-Alkoxy und/oder $C_1$-$C_4$-Alkylthio, eine $C_1$-$C_4$-Alkoxygruppe, eine $C_1$-$C_4$-Alkylthiogruppe, eine $C_3$-$C_6$-Alkenyloxygruppe oder eine $C_3$-$C_6$-Alkinyloxygruppe;

n 1, 2 oder 3 ;

sowie deren umweltverträglichen Salze, dadurch gekennzeichnet, daß man eine Verbindung der Formel II

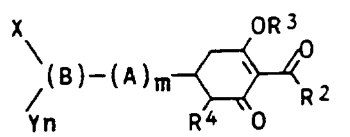

II

in an sich bekannter Weise

a) mit einem Hydroxylaminderivat der Formel $[R^1ONH_3]^{\oplus}Q^{\ominus}$, in der $Q^{\ominus}$ ein beliebiges Anion bedeutet, in einem inerten Lösungsmittel bei einer Temperatur zwischen 0 und 80 °C, in Gegenwart oder Abwesenheit einer Hilfsbase umsetzt oder

b) in einem inerten Lösungsmittel mit einem Hydroxylamin der Formel $R^1ONH_2$ bei Temperaturen zwischen 0 und 90 °C umsetzt.

**2.** Herbizides Mittel, enthaltend ein Cyclohexenonderivat der Formel I gemäß Anspruch 1 und inerte Zusatzstoffe

**3.** Herbizides Mittel nach Anspruch 2, enthaltend weitere wirksame Bestandteile.

**4.** Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Cyclohexenonderivats der Formel I gemäß Anspruch 1 behandelt.

**5.** Verwendung der Cyclohexenonderivate der Formel I gemäß Anspruch 1 als Herbizide.

## Claims

### Claims for the following Contracting States : BE, CH, DE, FR, GB, IT, LI, NL

1. A cyclohexanone derivative of the general formula I

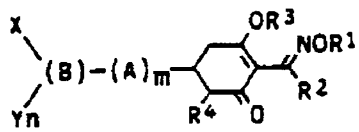

I

where

$R^1$ is $C_1$-$C_6$-alkyl, $C_3$-$C_6$-alkenyl or $C_3$-$C_6$-alkynyl and these groups may carry from one to three of the following radicals: halogen, $C_1$-$C_4$-alkoxy, $C_1$-$C_4$-alkylthio and/or phenyl, and the phenyl group in turn may be monosubstituted to trisubstituted by halogen, $C_1$-$C_4$-alkyl, $C_1$-$C_4$-alkoxy, $C_1$-$C_4$-alkylthio, $C_1$-$C_4$-haloalkyl and/or cyano;
phenyl, benzyl or thenyl, where the aromatic nuclei may carry from one to three of the following radicals: halogen, $C_1$-$C_4$-alkyl, $C_1$-$C_4$-alkoxy, $C_1$-$C_4$-alkylthio, $C_1$-$C_4$-haloalkyl, nitro and/or cyano;
$R^2$ is $C_1$-$C_6$-alkyl, $C_3$-$C_6$-alkenyl, $C_3$-$C_6$-alkynyl or phenyl, and these groups may carry from one to three of the following radicals: halogen, $C_1$-$C_4$-alkoxy, $C_1$-$C_4$-alkylthio, and/or cyano;
$R^3$ is hydrogen, $C_1$-$C_6$-alkyl, $C_1$-$C_{10}$-alkylcarbonyl, $C_1$-$C_6$-alkylsulfonyl, $C_1$-$C_4$-trialkylsilyl, $C_1$-$C_4$-dialkylphosphonyl, $C_1$-$C_4$-dialkylthiophosphonyl, $C_3$-$C_6$-alkenyl, $C_3$-$C_6$-alkenylcarbonyl or $C_3$-$C_6$-alkynyl or a benzoyl or phenylsulfonyl group which may be monosubstituted to trisubstituted by halogen, nitro, cyano, $C_1$-$C_4$-alkyl, $C_1$-$C_4$-haloalkyl, $C_1$-$C_4$-alkoxy and/or $C_1$-$C_4$-alkylthio;
$R^4$ is hydrogen, cyano, halogen, $C_1$-$C_6$-alkyl or $C_1$-$C_6$-alkoxycarbonyl;
A is a $C_1$-$C_4$-alkylene chain in which a methylene group may be replaced by an oxygen or sulfur atom and which may carry one or two of the following substituents: $C_1$-$C_4$-alkyl, $C_1$-$C_4$-alkoxy, $C_1$-$C_4$-alkylthio and/or halogen;
m is 0 or 1,
B is a 5-membered monounsaturated or diunsaturated heterocyclic radical having not more than 2 nitrogen atoms and/or not more than one oxygen or sulfur atom;
a 5-membered or 6-membered heteroaromatic radical, where these rings may carry from one to three of the following groups: $C_1$-$C_4$-alkyl, $C_1$-$C_4$-alkoxy, $C_1$-$C_4$-alkylthio, $C_1$-$C_4$-haloalkyl, halogen, cyano and/or nitro;
Y is hydrogen, halogen, cyano or nitro;

$C_1$-$C_6$-alkyl,

$C_2$-$C_6$-alkenyl or $C_2$-$C_6$-alkynyl, and these groups may carry from one to three of the following radicals: phenyl, halogen, $C_1$-$C_4$-alkoxy and/or $C_1$-$C_4$-alkylthio,
$C_1$-$C_4$-alkoxy, $C_1$-$C_4$-alkylthio, $C_3$-$C_6$-alkenyloxy or $C_3$-$C_6$-alkynyloxy; and
n is 1, 2 or 3;
or an environmentally compatible salt thereof.

2. A process for the preparation of a compound I as claimed in claim 1, wherein a compound of the formula II

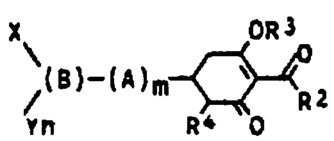

II

is reacted in a conventional manner with
a) a hydroxylamine derivative of the formula $[R^1ONH_3]^{\oplus}Q^{\ominus}$, where $Q^{\ominus}$ is any desired anion, in an inert solvent at from 0 to 80 °C, in the presence or absence of an auxiliary base, or

b) a hydroxylamine of the formula $R^1ONH_2$ in an inert solvent at from 0 to 90 °C.

**3.** A herbicide containing a cyclohexenone derivative of the formula I as claimed in claim 1 and inert additives.

**4.** A herbicide as claimed in claim 3, containing further active constituents.

**5.** A method for controlling undesirable plant growth, which comprises treating the undesirable plants and/or their habitat with a herbicidally effective amount of a cyclohexenone derivative of the formula I as claimed in claim 1.

**6.** The use of a cyclohexenone derivative of the formula I as claimed in claim 1 as a herbicide.

**Claims for the following Contracting State : ES**

**1.** A process for the preparation of a cyclohexanone derivative of the general formula I

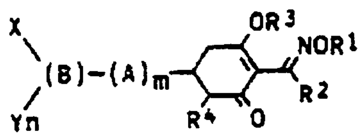

I

where
$R^1$ is $C_1$-$C_6$-alkyl, $C_3$-$C_6$-alkenyl or $C_3$-$C_6$-alkynyl and these groups may carry from one to three of the following radicals: halogen, $C_1$-$C_4$-alkoxy, $C_1$-$C_4$-alkylthio and/or phenyl, and the phenyl group in turn may be monosubstituted to trisubstituted by halogen, $C_1$-$C_4$-alkyl, $C_1$-$C_4$-alkoxy, $C_1$-$C_4$-alkylthio, $C_1$-$C_4$-haloalkyl and/or cyano;
phenyl, benzyl or thenyl, where the aromatic nuclei may carry from one to three of the following radicals: halogen, $C_1$-$C_4$-alkyl, $C_1$-$C_4$-alkoxy, $C_1$-$C_4$-alkylthio, $C_1$-$C_4$-haloalkyl, nitro and/or cyano;
$R^2$ is $C_1$-$C_6$-alkyl, $C_3$-$C_6$-alkenyl, $C_3$-$C_6$-alkynyl or phenyl, and these groups may carry from one to three of the following radicals: halogen, $C_1$-$C_4$-alkoxy, $C_1$-$C_4$-alkylthio, and/or cyano;
$R^3$ is hydrogen, $C_1$-$C_6$-alkyl, $C_1$-$C_{10}$-alkylcarbonyl, $C_1$-$C_6$-alkylsulfonyl, $C_1$-$C_4$-trialkylsilyl, $C_1$-$C_4$-dialkylphosphonyl, $C_1$-$C_4$-dialkylthiophosphonyl, $C_3$-$C_6$-alkenyl, $C_3$-$C_6$-alkenylcarbonyl or $C_3$-$C_6$-alkynyl or a benzoyl or phenylsulfonyl group which may be monosubstituted to trisubstituted by halogen, nitro, cyano, $C_1$-$C_4$-alkyl, $C_1$-$C_4$-haloalkyl, $C_1$-$C_4$-alkoxy and/or $C_1$-$C_4$-alkylthio;
$R^4$ is hydrogen, cyano, halogen, $C_1$-$C_6$-alkyl or $C_1$-$C_6$-alkoxycarbonyl;
A is a $C_1$-$C_4$-alkylene chain in which a methylene group may be replaced by an oxygen or sulfur atom and which may carry one or two of the following substituents: $C_1$-$C_4$-alkyl, $C_1$-$C_4$-alkoxy, $C_1$-$C_4$-alkylthio and/or halogen;
m is 0 or 1,
B is a 5-membered monounsaturated or diunsaturated heterocyclic radical having not more than 2 nitrogen atoms and/or not more than one oxygen or sulfur atom;
a 5-membered or 6-membered heteroaromatic radical, where these rings may carry from one to three of the following groups: $C_1$-$C_4$-alkyl, $C_1$-$C_4$-alkoxy, $C_1$-$C_4$-alkylthio, $C_1$-$C_4$-haloalkyl, halogen, cyano and/or nitro;
Y is hydrogen, halogen, cyano or nitro;

$C_1$-$C_6$-alkyl,

$C_2$-$C_6$-alkenyl or $C_2$-$C_6$-alkynyl, and these groups may carry from one to three of the following radicals: phenyl, halogen, $C_1$-$C_4$-alkoxy and/or $C_1$-$C_4$-alkylthio,
$C_1$-$C_4$-alkoxy, $C_1$-$C_4$-alkylthio, $C_3$-$C_6$-alkenyloxy or $C_3$-$C_6$-alkynyloxy; and
n is 1, 2 or 3;
or an environmentally compatible salt thereof, wherein a compound of the formula II

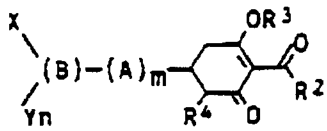

II

is reacted in a conventional manner with

a) a hydroxylamine derivative of the formula $[R^1ONH_3]^{\oplus}Q^{\ominus}$, where $Q^{\ominus}$ is any desired anion, in an inert solvent at from 0 to 80 °C, in the presence or absence of an auxiliary base, or

b) a hydroxylamine of the formula $R^1ONH_2$ in an inert solvent at from 0 to 90 °C.

**2.** A herbicide containing a cyclohexenone derivative of the formula I as claimed in claim 1 and inert additives.

**3.** A herbicide as claimed in claim 2, containing further active constituents.

**4.** A method for controlling undesirable plant growth, which comprises treating the undesirable plants and/or their habitat with a herbicidally effective amount of a cyclohexenone derivative of the formula I as claimed in claim 1.

**5.** The use of a cyclohexenone derivative of the formula I as claimed in claim 1 as a herbicide.

**Revendications**

**Revendications pour les Etats contractants suivants : BE, CH, DE, FR, GB, IT, LI, NL**

**1.** Dérivés de cyclohexenone de la formule générale I

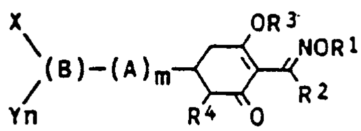

I

dans laquelle les substituants ont les significations suivantes

$R^1$

- un groupe alkyle en C1-C6, un groupe alcenyle en C3-C6 ou un groupe alcinyle en C3-C6, ces groupes pouvant porter un à trois des restes suivants : halogène, alcoxy en C1-C4, alkylthio en C1-C4 et/ou phenyle, le groupe phenyle pouvant de son côté être substitué jusqu'à trois fois par halogène, alkyle en C1-C4, alcoxy en C1-C4, alkylthio en C1-C4, halogenalkyle en C1-C4, et/ou cyano
- un groupe phenyle, un groupe benzyle ou un groupe thenyle, les noyaux aromatiques pouvant porter un à trois des restes suivants : halogène, alkyle en C1-C4, alcoxy en C1-C4, alkylthio en C1-C4, halogenalkyle en C1-C4, et/ou cyano

$R^2$ un groupe alkyle en C1-C6, alcenyle en C3-C6, alcinyle en C3-C6 ou phenyle, ces groupes pouvant porter un à trois des restes suivants : halogene, alcoxy en C1-C4, alkylthio en C1-C4 et/ou cyano

R3 hydrogène, un groupe alkyle en C1-C6, (alkyle en C1-C10)-carbonyle, (alkyle en C1-C6) sulfonyle, (trialkyle en C1-C4)-silyle, (dialkyle en C1-C4) phosphonyle, (dialkyle en C1-C4) thiophosphonyle, (alcenyle en C3-C6), (alcenyle en C3-C6)-carbonyle, alcinyle en C3-C6 ou un groupe benzoyle ou phenylsulfonyle, qui peuvent être substitués une à trois fois par halogene, nitro, cyano, alkyle en C1-C4, halogenalkyle en C1-C4, alcoxy en C1-C4, et/ou alkylthio en C1-C4.

R4 hydrogène, un groupe cyano, un atome d'halogène, un groupe alkyle en C1-C6 ou un groupe (alcoxy en C1-C6)-carbonyle

A une chaîne alkylene en C1-C4 dans laquelle un groupe methylene peut être remplacé par un atome d'oxygène ou de soufre et qui peut porter un à trois des substituants suivants : alkyle en C1-C4, alcoxy en C1-C4, alkythio en C1-C4 et/ou halogène

m 0 ou 1

B un hétérocycle à 5 chaînons, insaturé une ou deux fois, ayant jusqu'à 2 atomes d'azote et/ou jusqu'à un atome d'oxygène ou soufre

X un hétéroaromate à 5-6 chaînons, ces cycles pouvant porter un à trois des groupes suivants : alkyle en C1-C4, alcoxy en C1-C4, alkylthio en C1-C4, halogenalkyle en C1-C4, halogene, cyano et/ou nitro

Y hydrogène, un atome d'halogène, un groupe cyano, un groupe nitro, un groupe alkyle en C1-C6, un groupe alcényle en C2-C6 ou un groupe alcinyle en C2-C6, ces groupes pouvant porter un à trois des restes suivants : phényle, halogène, alcoxy en C1-C4, un groupe alkylthio en C1-C4, un groupe alcényloxy en C3-C6 ou un groupe alcinyloxy en C3-C6.

n 1, 2 ou 3

ainsi que leurs sels acceptables pour l'environnement.

**2.** Procédé de préparation des composés I selon la revendication 1, caractérisé en ce qu'on fait réagir un composé de la formule II

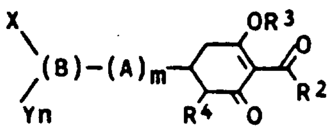

II

de manière connue en soi

a) avec un dérivé d'hydroxylamine de la formule $[R^1ONH_3]^{\oplus}Q^{\ominus}$ dans laquelle $Q^{\ominus}$représente un anion arbitraire, dans un solvant inerte, à une température entre 0 et 80 ° C, en présence ou absence d'une base auxiliaire, ou

b) dans un solvant inerte avec une hydroxylamine de la formule $R^1$ $ONH_2$ à des températures entre 0 et 90 ° C

**3.** Agent herbicide contenant un dérivé de cyclohexenone de la formule I selon la revendication 1 et des additifs inertes.

**4.** Agent herbicide selon la revendication 3 contenant d'autres constituants actifs.

**5.** Procédé pour lutter contre une croissance de plantes indésirable, caractérisé en ce que l'on traite les plantes indésirables et/ou leur biotope avec une quantité efficace comme herbicide d'un dérivé de cyclohexenone de formule I selon la revendication 1.

**6.** Utilisation des dérivés de cyclohenenone de la formule I selon la revendication 1, comme herbicides.

**Revendications pour l'Etat contractant suivant : ES**

**1.** Procédé de préparation de dérivés de cyclohexenone de la formule générale

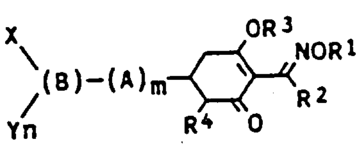

I

dans laquelle les substituants ont les significations suivantes

$R^1$

- un groupe alkyle en C1-C6, un groupe alcenyle en C3-C6 ou un groupe alcinyle en C3-C6, ces groupes pouvant porter un à trois des restes suivants : halogène, alcoxy en C1-C4, alkylthio en C1-C4 et/ou phenyle, le groupe phenyle pouvant de son côté être substitué jusqu'à trois fois par halogène, alkyle en C1-C4, alcoxy en C1-C4, alkylthio en

C1-C4, halogenalkyle en C1-C4, et/ou cyano
- un groupe phenyle, un groupe benzyle ou un groupe thenyle, les noyaux aromatiques pouvant porter un à trois des restes suivants : halogène, alkyle C1-C4, alcoxy C1-C4, alkylthio C1-C4, halogenalkyle C1-C4, et/ou cyano

$R^2$ un groupe alkyle en C1-C6, alcenyle en C3-C6, alcinyle en C3-C6 ou phenyle, ces groupes pouvant porter un à trois des restes suivants : halogene, alcoxy en C1-C4, alkylthio en C1-C4 et/ou cyano

R3 hydrogène, un groupe alkyle en C1-C6, (alkyleen C1-C10)-carbonyle, (alkyle en C1-C6) sulfonyle, (trialkyle en C1-C4)-silyle, (dialkyle en C1-C4) phosphonyle, (dialkyle en C1-C4) thiophosphonyle, (alcenyle en C3-C6), (alcenyle en C3-C6)-carbonyle, alcinyle en C3-C6 ou un groupe benzoyle ou phenylsulfonyle, qui peuvent être substitués une à trois fois par halogene, nitro, cyano, alkyle en C1-C4, halogenalkyle en C1-C4, alcoxy en C1-C4, et/ou alkylthio en C1-C4.

R4 hydrogène, un groupe cyano, un atome d'halogène, un groupe alkyle en C1-C6 ou un groupe (alcoxy en C1-C6)-carbonyle

A une chaîne alkylene en C1-C4 dans laquelle un groupe methylene peut être remplacé par un atome d'oxygène ou de soufre et qui peut porter un à trois des substituants suivants : alkyle en C1-C4, alcoxy en C1-C4, alkythio en C1-C4 et/ou halogène

m 0 ou 1

B un hétérocycle à 5 chaînons, insaturé une ou deux fois, ayant jusqu'à 2 atomes d'azote et/ou jusqu'à un atome d'oxygène ou soufre

X un hétéroaromate à 5-6 chaînons
ces cycles pouvant porter un à trois des groupes suivants : alkyle en C1-C4, alcoxy en C1-C4, alkylthio en C1-C4, halogenalkyle en C1-C4, halogene, cyano et/ou nitro

Y hydrogène, un atome d'halogène, un groupe cyano, un groupe nitro, un groupe alkyle en C1-C6, un groupe alcényle en C2-C6 ou un groupe alcinyle en C2-C6, ces groupes pouvant porter un à trois des restes suivants : phényle, halogène, alcoxy en C1-C4, un groupe alkylthio en C1-C4, un groupe alcényloxy en C3-C6 ou un groupe alcinyloxy en C3-C6.

n 1, 2 ou 3

ainsi que leurs sels acceptables pour l'environnement, caractérisé par le fait qu'on fait réagir un composé de la formule II

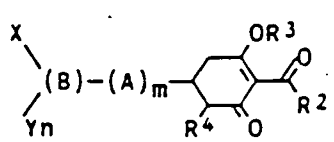

II

de manière connue en soi

a) avec un dérivé d'hydroxylamine de la formule $[R^1ONH_3]^{\oplus}Q^{\ominus}$ dans laquelle $Q^{\ominus}$représente un anion arbitraire, dans un solvant inerte, à une température entre 0 et 80 °C, en présence ou absence d'une base auxiliaire, ou

b) dans un solvant inerte avec une hydroxylamine de la formule $R^1ONH_2$ à des températures entre 0 et 90 °C

**2.** Agent herbicide contenant un dérivé de cyclohexenone de la formule I selon la revendication 1 et des additifs inertes.

**3.** Agent herbicide selon la revendication 2 contenant d'autres constituants actifs.

**4.** Procédé pour lutter contre une croissance de plantes indésirables, caractérisé en ce que l'on traite les plantes indésirables et/ou leur biotope avec une quantité efficace comme herbicide d'un dérivé de cyclohexenone de formule I selon la revendication 1.

**5.** Utilisation des dérivés de cyclohexenone de la formule I selon la revendication 1, comme herbicides.